# FASCICULE DE BREVET EUROPEEN

(11) **EP 1 413 286 B1**
(45) Date de publication et mention de la délivrance du brevet: **22.04.2009**
(21) Numéro de dépôt: 03292454.0
(22) Date de dépôt: 03.10.2003
(51) Int. Cl.: A61Q 5/10, A61K 8/49

(54) **Composition tinctoriale comprenant au moins une base d'oxydation hétérocyclique et au moins un coupleur 2,3-diaminopyridine substitué**
Färbemittel enthaltend mindestens ein heterocyclisches Oxidationsmittel und mindestens eine substituierten 2,3-Diaminopyridin-Kupplungsmittel
Dyeing composition comprising at least one heterocyclic oxidation base and at least one substituted 2,3-diaminopyridine as a coupling agent

(30) Priorité: 04.10.2002 FR 0212360
(43) Date de publication de la demande: 28.04.2004
(73) Titulaire: L'ORÉAL, 75008 Paris (FR)
(72) Inventeur: Kravtchenko, Sylvain, 92600 Asnieres (FR); Lagrange, Alain, 77700 Coupvray (FR); Fadli, Aziz, 77500 Chelles (FR)
(74) Mandataire: Wattremez, Catherine

(56) Documents cités:
- EP-A- 1 336 606
- WO-A-02/055500
- WO-A-02/076418
- DE-A- 4 115 148
- DE-A- 19 927 074
- DE-U- 20 106 648
- FR-A- 2 779 952
- US-A1- 2002 013 973

## Description

L'invention a pour objet une composition tinctoriale comprenant au moins une base d'oxydation hétérocyclique et au moins un coupleur du type 2,3-diaminopyridine substitué. L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine ou entre des cheveux différemment sensibilisés au sein d'une même chevelure.

Il est déjà connu d'utiliser des compositions tinctoriales comprenant en tant que base d'oxydation hétéroaromatique pour la teinture de fibres kératiniques. Par exemple, le brevet US 5 380 340 décrit des bases d'oxydation du type pyrazolopyrimidine. La demande de brevet FR 2 750 048 décrit des compositions pour la teinture d'oxydation des fibres kératiniques comprenant une base d'oxydation du type pyrazolo-[1,5-a]-pyrimidine. Ces bases d'oxydation permettent d'obtenir des nuances variées, en particulier dans le domaine des rouges. Cependant, elles ne permettent pas d'obtenir des nuances foncées, en particulier des nuances grises à noires.

Par ailleurs, il est connu d'utiliser dans le domaine de la coloration des fibres kératiniques des coupleurs pyridines. Le document US 4 784 667 décrit en particulier des coupleurs 2,3-diamino 6-méthoxypyridine. Ces coupleurs lorsqu'ils sont utilisés avec des bases benzéniques du type diamine aromatique, 2-aminophenol ou 4-aminophénol permettent d'obtenir des couleurs intenses et stables dans les nuances bleues.

La demande de brevet FR 2779952 décrit des compositions tinctoriales contenant à titre de coupleur un coupleur pyridinique et à titre de base une base pyrazolopyrimidine. Les colorations qui sont ainsi obtenues ne sont pas suffisamment neutres et puissantes.

Le document DE 4 115 148 décrit des compositions comprenant un coupleur 6-alcoxy 2,3-diaminopyridine avec des bases triaminopyrimidiniques. Les colorations qui sont ainsi obtenues ne sont pas suffisamment neutres et puissantes.

Le but de la présente invention est de développer de nouvelles compositions tinctoriales permettant d'obtenir une coloration des fibres kératiniques dans des nuances sombres et peu chromatiques, en particulier des nuances variant du gris au noir, la coloration étant peu sélective et présentant une bonne ténacité vis à vis des différentes agressions extérieures que peuvent subir les cheveux.

Ce but est atteint avec la présente invention qui a pour objet une composition tinctoriale comprenant, dans un milieu de teinture approprié,
- au moins un coupleur de formule (I) 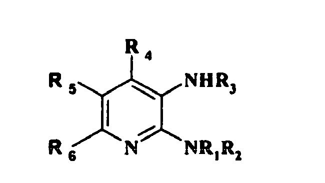 dans laquelle
   -R3 et R4 représentent, indépendamment un atome d'hydrogène ; un radical alkyle linéaire ou ramifié de 1 à 6 atomes de carbone, éventuellement substitué par un ou plusieurs atomes d'halogène, un ou plusieurs radicaux hydroxy, amino, mono, di ou tri alkyle(C1-C4)amino, aryle, alcoxyC₁-C₃ ou hétérocycle ; un radical arylC₆-C₁₈, éventuellement substitué par un ou plusieurs radicaux amino, hydroxy, alcoxy(C₁-C₃) ou alkyl(C₁-C₆) ; un radical alcényle en
   C₂-C₆ ; un radical carboxyalkyl(C₁-C₆) ou un radical alkylthio(C₁-C₆) ; et
   - R₁ et R₂, ensemble, forment avec l'atome d'azote auquel ils sont rattachés un cycle pyrrolidine, piperidine, hormopiperidine, imidazoline, pyrozolidine, piperazine
   - R₅ représente un atome d'hydrogène ; un radical hydroxy ; alcoxy ; aryloxy ; un atome d'halogène ; un radical -OSO₂R ; un radical -OCOR ; un radical trifluoroalcoxy en C₁-C₃ ;
   - R6 représente un radical hydroxy ; OR ; aryloxy ; un atome d'halogène ; un radical -OSO₂R ; un radical -OCOR ; un radical trifluoroalcoxy en C₁-C₃ ;
   - R représente un radical alkyle en C₁-C₈ pouvant être substitué par un radical hydroxy, amino ou alcoxy en C₁-C₄ ;
- au moins une base d'oxydation hétérocyclique choisie parmi les pyridines non condensés, les pyrazoles non condensés, les pyrazolopyrimidines condensées, les pyrazolotriazoles, les pyrazolotétrazole, les pyrazolopyridazines, les pyrazolothiazoles, les pyrazoloimidazoles, les pyrazoloquinolines, les pyrazolopyrazoles, les pyrazolobenzimidazoles, les aminopyrazolones, les aminopyrolidines, les aminopyrazolines, les monoaminoquinoleines, les aminoindazoles, les diamino-uraciles, les hydrazones, les aminoindolénines, les diamino ou triaminoquinoleines, les dérivés de julolidine ou de lilolidine.

L'invention a aussi pour objet un procédé de teinture des fibres kératiniques à partir de cette composition.

Un autre objet de l'invention est l'utilisation de la composition de la présente invention pour la teinture des fibres kératiniques, en particulier les fibres kératiniques humaines telles que les cheveux.

La composition de la présente invention permet en particulier d'obtenir une coloration de fibres kératiniques sans virage de la couleur après lavage. En effet, même si la couleur après différents lavages, a tendance à s'estomper en intensité, il est particulièrement intéressant de ne pas modifier par ces lavages la composante reflet de la nuance initiale.

Dans le cadre de la présente invention, on entend par alkyle linéaires des radicaux tels que méthyle, éthyle, n-propyle, n-butyle et par alkyle ramifié des radicaux iso-propyle, iso-butyle, tertio-butyle, etc.

Un radical aryle en C₈-C₁₈ est un radical par exemple choisi parmi un radical phényle, un radical benzyle, un radical naphtyle.

Par le terme "non condensé", on entend au sens de la présente invention les monocycles isolés éventuellement sous forme de dimère.

A titre de diamino-uraciles , on peut citer les composés 5,6-diamino - uracile, 5,6-diamino-2-thio -uracile, 5,6-diamino-3-méthyl -uracile, 5-amino-3-méthyl-6-méthylamino -uracile, 5-amino-3-méthyl-6-bétahydroxyéthylamino -uracile, 5-amino-3-méthyl-6-benzylamino -uracile, 5-amino-3-méthyl-6-phénylamino -uracile, 5,6-diamino-1-phényl-uracile, 5,6-diamino-1,3-diméthyl -uracile, 5-amino-1,3-diméthyl-6-méthylamino -uracile, 5-amino-1,3-diméthyl-6-bétahydroxyméthylamino -uracile, 5-amino-1,3-diméthyl-6-benzyllamino -uracile, 5-amino-1,3-diméthyl-6-phényllaminouracile, 5-amino-1,3-diméthyl-6-diméthylamino -uracile. De tels composés sont décrits dans le document DE 2533629.

A titre d'aminoindolénines, on peut citer les composés 2-méthyl-5-aminoindolénine, 1-bétahydroxyéthyl-2-méthyl-5-aminoindolénine. De tels composés sont décrits dans le document FR 1602547.

A titre de mono ou diaminotétrahydroquinoléines, on peut citer les composés 5-amino-1,2,3,4-tétrahydroquinoléine, 5-amino-7-chloro-8-pipéridino-1,2,3,4-tétrahydroquinoléine, 5-amino-7-chloro-8-morpholino-1,2,3,4-tétrahydroquinoléine, 5,7-diamino-6-méthyl-8-hydroxy-1,2,3,4-tétrahydroquinoléine, 5-amino-8-méthoxy-1,2,3,4-tétrahydroquinoléine, 5-amino-7-chloro-8-diméthylamino-1,2,3,4-tétrahydroquinoléine. De tels composés sont décrits dans le document DE 2441895.

A titre de diaminoquinoléines, on peut citer les composés 5,7-diamino-6-méthyl-8-hydroxyquinoléine et 5,7-diamino-2-méthyl-8-hydroxyquinoléine. De tels composés sont décrits dans le document DE 2441598.

A titre de triaminoquinoléine, on peut citer 5,7-diamino-8-méthylaminoquinoléine, 5,7-diamino-8-diméthylaminoquinoléine, 5,7-diamino-8-morpholinoquinoléine, 5,7-diamino-8-bétahydroxyéthylaminoquinoléine, 5,7,8-triaminoquinoléine. De tels composés sont décrits dans le document DE 2441599.

A titre d'aminopyrazolones, on peut citer l'acide 1-phényl-4-aminopyrazolone-3-carbonique, l'amide de l'acide 1-phényl-4-aminopyrazolone-3-carbonique, l'ester éthylique de l'acide 1-phényl-4-aminopyrazolone-3-carbonique, la 1-phényl-3-méthyl-4-aminopyrazolone. De tels composés sont décrits dans le document FR 1488169. On peut aussi citer la 3-amino-pyrazolone, 1-phényl-3-amino-pyrazolone, 1-phényl-3-hydroxy-pyrazolone. De tels composés sont décrits dans le document FR 1564999.

A titre de pyrazolothiazoles, on peut citer les composés 3-amino-2-méthyl-pyrazolo[3,2-b]thiazole, 3-amino-pyrazolo[3,2-b]thiazole, 3-amino-2,5-diméthyl-6-phénylpyrazolo[3,2-b]thiazole. De tels composés sont décrits dans le document US 5 427 200.

A titre de pyrazolotriazoles, on peut citer les composés 3-amino-4-méthyl-6-méthylthio-2-phényl-pyrazolo[3,2-c]-s-triazole, 3-amino-2,4,6-triméthyl-pyrazolo[3,2-c]-s-triazole, 3-amino-4,6-diméthyl-pyrazolo[3,2-c]-s-triazole. De tels composés sont décrits dans le document US 5 457 200. On peut aussi citer les composés 7-amino-2,6-diméthyl-pyrazolo[1,5-b]-1,2,4-triazole, 7-amino-3,6-diméthyl-pyrazolo[3,2-c]-1,2,4-triazole, 7-amino-3-méthyl-pyrazolo[3,2-c]-1,2,4-triazole, 7-amino-3-méthyl-6-carboxypyrazolo[3,2-c]-1,2,4-triazole, 7-amino-2-méthyl-pyrazolo[1,5-b]-1,2,4-triazole 7-amino-2-phényl-pyrazolo[1,5-b]-1,2,4-triazole, 7-amino-2-méthyl-6-carboxy-pyrazolo[1,5-b]-1,2,4-triazole. Ces composés sont décrits dans le EP 923929.

A titre de pyrazoloimidazoles, on peut citer les composés 3-amino-4-benzyl-pyrazolo[1,5-a]imidazole, 3-amino-2,4-diméthyl-pyrazolo[1,5-a]imidazole, 3-amino-4-méthyl-pyrazolo[1,5-a]imidazole. De tels composés sont décrits dans le document US 5457200. On peut aussi citer les composés 7-amino-6-méthyl-pyrazolo[1,5-a]imidazole, 7-amino-pyrazolo[1,5-a]imidazole, 7-amino-2-méthyl-pyrazolo[1,5-a]imidazole, 7-amino-2-phényl-pyrazolo[1,5-a]imidazole décrits dans le EP 923929.

A titre de pyrazoloquinolines, on peut citer les composés 3-amino-2-phényl-pyrazolo[1,5-a]quinoline. De tels composés sont décrits dans le document US 5 457200.

A titre de pyrazolopyridazine, on peut citer la 3-amino-pyrazolo[1,5-b]pyridazine. De tels composés sont décrits dans le document US 5 457200.

A titre de dérivés de julolidine ou de lilolidine, on peut citer les composés 9-aminojulolidine, 9-amino-8-méthyljulolidine, 9-amino-8,10-diméthyljulolidine, 8-aminolilolidine. De tels composés sont décrits dans le document DE 2441597.

A titre d'hydrazone, on peut citer les composés N-méthyl-pyridone-4-hydrazone, N-méthyl-thiazolone-hydrazone, N-méthyl-thiazolone-2-hydrazone, N,N-diméthyl-benzimidazolone-hydrazone, N-méthyl-pyridone-2-hydrazone, N-méthyl-benzothiazolone-2-hydrazone, 1,2-diméthyl-indazolone-3-hydrazone, 1,2,6-triméthyl-pyridone-4-hydrazone, 1-méthyl-quinolone-2-hydrazone, 1,2,6-triméthyl-3-nitropyridone-4-hydrazone, 1,2,6-triméthyl-3-aminopyridone-4-hydrazone, N-méthyl-cyclohexènothiazolone-hydrazone, 1,2,5-triméthyl-pyrazolone-3-hydrazone, 1,2-diméthyl-indazolone-3-hydrazone, 1,2-diméthyl-5-chloro-indazolone-3-hydrazone, 1-méthyl-2-éthyl-5-nitro-indazolone-3-hydrazone, N-méthyl-quinolone-4-hydrazone, N-méthyl-benzothiazolone-2-omégabenzènesulfonyl-hydrazone. De tels composés sont décrits dans le document FR 1 602 547.

A titre d'aminopyrazolines, on peut citer les composés 1-(4'-aminophényl)-3-aminopyrazoline, 1-(4'-hydroxyphényl)-3-aminopyrazoline. De tels composés sont décrits dans le document FR 2018056.

A titre d'aminoindazoles, on peut citer 4,7-diamino-5-méthylindazole, 4,7-diamino-5,6-diméthylindazole, 6,7-diaminoindazole, 6-hydroxy-7-aminoindazole, 1-éthyl-6-hydroxy-7-aminoindazole, 6-aminoindazole, 5,6-diaminoindazole. De tels composés sont décrits dans le document FR 2315906, DE 1492166.

A titre de pyrazolobenzimidazoles, on peut citer les composés 7-amino-6-méthyl-pyrazolo[1,5-a]benzimidazole, 6,7-diamino-pyrazolo[1,5-a]benzimidazole, 6,7-diamino-2-méthyl-pyrazolo[1,5-a]benzimidazole, 6,7-diamino-2-phényl-pyrazolo[1,5-a]benzimidazole décrits par exemple dans le EP 923929.

A titre de pyrazolotétrazoles, on peut citer les composés 7-amino-6-méthyl-pyrazolo[1,5-e]tétrazole, 7-amino-6-phényl-pyrazolo[1,5-e]tétrazole, 7-amino-6-carboxy-pyrazolo[1,5-e]tétrazole décrits dans le EP 923929.

Parmi les bases d'oxydation hétérocycliques citées précédemment, on préfère les pyridines non condensées, les pyrazoles non condensées, les pyrazolopyrimidines, les pyrazolotriazoles.

De préférence, ces bases sont choisies parmi les composés 2,5-diamino pyridine, le 4,5-diamino 1-méthyl pyrazole, 4,5-diamino 1-(β-hydroxyéthyl) pyrazole, 3,4-diamino pyrazole, 4,5-diamino 1-(4'-chlorobenzyl) pyrazole, 4,5-diamino 1,3-diméthyl pyrazole, 4,5-diamino 3-méthyl 1-phényl pyrazole, 4,5-diamino 1-méthyl 3-phényl pyrazole, 4-amino 1,3-diméthyl 5-hydrazino pyrazole, 1-benzyl 4,5-diamino 3-méthyl pyrazole, 4,5-diamino 3-tert-butyl 1-méthyl pyrazole, 4,5-diamino 1-tert-butyl 3-méthyl pyrazole, 4,5-diamino 1-(β-hydroxyéthyl) 3-méthyl pyrazole, 4,5-diamino 1-éthyl 3-méthyl pyrazole, 4,5-diamino 1-éthyl 3-(4'-méthoxyphényl) pyrazole, 4,5-diamino 1-éthyl 3-hydroxyméthyl pyrazole, 4,5-diamino 3-hydroxyméthyl 1-méthyl pyrazole, 4,5-diamino 3-hydroxyméthyl 1-isopropyl pyrazole, 4,5-diamino 3-méthyl 1-isopropyl pyrazole, 4-amino 5-(2'-aminoéthyl)amino 1,3-diméthyl pyrazole, 3,4,5-triamino pyrazole, 1-méthyl 3,4,5-triamino pyrazole, 3,5-diamino 1-méthyl 4-méthylamino pyrazole, 3,5-diamino 4-(β-hydroxyéthyl)amino 1-méthyl pyrazole, 1,3-bis-(4,5-diaminopyrazol-1-yl)propane, 1,3-bis-(4,5-diaminopyrazol-1-yl)propane-2-amine, 2, 2'-bis-(4,5-diaminopyrazol-1-yl)diéthylamine, 1,3-bis-(4,5-diaminopyrazol-1-yl)propane-2-ol, 1,3-bis-(4,5-diaminopyrazol-1-yl)propane-2-ol, 2, 2'-bis-(4,5-diaminopyrazol-1-yl)diéthylether, 2, 2'-bis-(4,5-diaminopyrazol-1-yl)diéthylthioéther, 2, 2'-bis-(4,5-diaminopyrazol-1-yl)diéthylméthylamine, 2, 2'-bis-(4,5-diaminopyrazol-1-yl)diéthyldiméthylamonium, la 7-amino 3-méthylpyrazole[3,2,c]-1,2,4-triazole, la 7-amino 2-méthyl pyrazolo [1,5-b]1,2,4-triazole, la pyrazolo-[1,5-a]-pyrimidine-3,7-diamine ; la 2,5-diméthyl pyrazolo-[1,5-a]-pyrimidine-3,7-diamine ; la 3-amino-7-β-hydroxyéthylamino-5-méthyl-pyrazolo-[1,5-a]-pyùmidine; le 2-(7-amino pyrazolo-[1,5-a]-pyrimidin-3-ylamino)-éthanol ; la 5,6-diméthyl pyrazolo-[1,5-a]-pyrimidine-3,7-diamine ; la 2, 5, N-7, N-7-tétraméthyl pyrazolo-[1,5-a]-pyrimidine-3,7-diamine ; la N-7-méthyl N-7-éthyl pyrazolo[1,5-a]-pyrimidine-3,7-diamine et leurs sels d'addition et leurs formes tautomères, lorsqu'il existe un équilibre tautomérique

Le coupleur utile dans la présente invention est de préférence représenté par la formule (l') 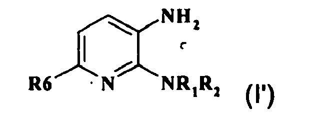 dans laquelle R₁, R₂ et R₆ sont tels que définis précédemment.
Selon un mode de réalisation particulièrement préféré, R6 est OR.

R1 et R2 peuvent former ensemble avec l'atome d'azote auquel ils sont attachés un cycle pyrrolidine, pipéridine, homopipéridine, imidazoline, pyrazolidine, piperazine.

A titre d'exemple, le coupleur pyridinique est choisi parmi les composés 6-méthoxy 3-amino 2-pyrrolidinylpyridine, 6-méthoxy 3-amino 2-(3'acétamidopyrrolidinyl)pyridine, 6-méthoxy 3-amino 2-(2'5'diméthylpyrrolidinyl)pyridine, 6-méthoxy 3-amino 2-morpholinopyridine, 6-méthoxy 3-amino 2-(2'-méthylpyrrolidinyl)pyridine, 6-méthoxy 3-amino 2-pipérazinylpyridine, 6-méthoxy 3-amino 2-pyridinylpyridine, 6-méthoxy 3-amino 2-pyrrolidinylpyridine, 6-méthoxy 3-amino 2-(2'-méthylpyridinyl)pyridine, 6-méthoxy 3-amino 2-(2'-hydroxyéthylpyridinyl)pyridine, 6-méthoxy 3-amino 2-(2'-pyrrolidinyléthyl)aminopyridine, 6-méthoxy 3-amino 2-(3'imidazolinylpropyl)aminopyridine, 6-méthoxy 3-amino 2-[3'-(3"-méthylimidazolium)propyl)aminopyridine,

De préférence, le coupleur pyridinique est choisi parmi les composés 6-méthoxy 3-amino 2-pyrrolidinylpyridine, 6-méthoxy 3-amino 2-(2'-méthylpyrrolidinyl)pyridine, 6-méthoxy 3-amino 2-(2'-méthylpyridinyl)pyridine, 6-méthoxy 3-amino 2-(2'-hydroyéthylpyridinyl)pyridine.

Ces coupleurs peuvent être peuvent être préparés selon des méthodes connues et décrites dans la littérature. On pourra se reporter à titre d'exemples au DE 3233540.

Une composition tinctoriale pour la teinture des fibres kératiniques peut en outre comprendre une ou plusieurs bases d'oxydation classiques dans le domaine de la coloration. A titre d'exemple, on peut citer les paraphénylènediamines, les bis-phénylalkylènediamines, les para-aminophénols, les ortho-aminophénols, les bases hétérocycliques autres que celles décrites précédemment et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-p-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino 2-chloro phénol, le 4-amino 2,6 dichlorophénol et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

La composition de la présente invention peut de plus contenir des bases hétérocycliques autres que celles utiles dans la présente invention.

La ou les bases d'oxydation présentes dans la composition de l'invention sont en général présentent chacune en quantité comprise entre 0,001 et 10 % en poids environ du poids total de la composition tinctoriale, de préférence entre 0,005 et 6 %.

La composition selon l'invention peut contenir un ou plusieurs coupleurs conventionnellement utilisés pour la teinture de fibres kératiniques autres que le coupleur pyridinique utile dans la présente invention. Parmi ces coupleurs, on peut notamment citer les métaphénylènediamines, les méta-aminophénols, les métadiphénols, les coupleurs naphtaléniques, les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylénedioxybenzéne, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à invention ne soient pas, ou substantiellement pas, altérées par la ou les adjonctions envisagées.

Le pH de la composition tinctoriale conforme à l'invention est généralement compris entre 3 et 12 environ, et de préférence entre 4 et 11 environ. Selon un mode de réalisation préféré, le pH est compris entre 5 et 8. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 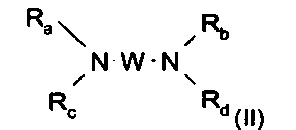 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Selon un premier mode de réalisation, le procédé de teinture des fibres kératiniques de la présente invention est un procédé dans lequel on applique sur les fibres la composition selon la présente invention telle que définie précédemment, en présence d'un agent oxydant pendant un temps suffisant pour développer la couleur désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. Le pH de la composition prêt à l'emploi est de préférence inférieur à 9, et encore plus préférentiellement inférieur ou égal à 7.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré. L'oxygène de l'air peut aussi être utilisé comme agent oxydant.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante prête à l'emploi appliquée sur les fibres kératiniques est de préférence inférieur à 9, de préférence encore inférieur ou égal à 7, et plus préférentiellement compris entre 5 et 7. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un second mode de réalisation du procédé de l'invention consiste à appliquer la composition de invention sur les cheveux et de laisser agir l'oxygène de l'air pendant un temps suffisant pour atteindre la coloration souhaitée.

L'invention a aussi pour objet un kit pour la coloration des cheveux comprenant d'une part une composition tinctoriale telle que défini précédemment et d'autre part une composition contenant un agent oxydant, ainsi qu'un dispositif à plusieurs compartiments dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### EXEMPLES DE TEINTURE

On a préparé la composition de teinture, conforme à l'invention, suivante :
- 5-méthyl-3,7-diaminopyrazolopyrimidine 0,76g
- 6-méthoxy 3-amino 2-[3'-(3"-méthylimidazolium)propyl)aminopyridine 1,11g
- Alcool oléique polyglycérolé à 4 moles de glycérol, à 78 % de matières actives (M.A.) 5,7 g M.A.
- Alcool oléique polyglycérolé à 2 moles de glycérol 4,0 g
- Acide oléique 3,0 g
- Amine oléique à 2 moles d'oxyde d'éthylène, vendue sous la dénomination Ethomeen 012 par la Société AKZO 7,0 g
- Laurylamino succinamate de diéthylaminopropyle, sel de sodium, à 55 % de M.A. 3,0 g M.A.
- Alcoololéique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse à 35 % de M.A. 0,46 g M.A.
- Acétate d'ammonium 0,8 g
- Antioxydant, séquestrant, q.s.
- Parfum, conservateur, q.s.
- Eau déminéralisée q.s.p. 100,0g

Au moment de l'emploi, on a mélangé cette composition poids pour poids avec de l'eau oxygénée titrant 20 volumes (6% en poids), de pH 2. Le pH du mélange est de 7.

On applique ce mélange sur des cheveux gris naturels à 90 % de blancs et sur des cheveux gris naturels à 90 % de blancs permanentés pendant 30 minutes. Après rinçage, lavage au shampooing, rinçage et séchage, les cheveux sont teints.

Chaque mèche est évaluée dans le système L*a*b*, au moyen d'un spectrocolorimètre CM 2002 MINOLTA ®, (Illuminant D65).

Dans le système L* a* b*, les trois paramètres désignent respectivement l'intensité (L*), la nuance (a*) et la saturation (b*). Selon ce système, plus la valeur de L est élevée, plus la couleur est claire ou peu intense. Inversement, plus la valeur de L est faible, plus la couleur est foncée ou très intense a* et b* indiquent deux axes de couleurs, a* indique l'axe de couleur vert/rouge et b* l'axe de couleur bleu/jaune.

| | **Reflet (notation visuelle)** | **Mesures colorimétriques prise au spectrocoelorimètre Minolta CM2002** | | |
|---|---|---|---|---|
| | | L* | a* | b* |
| **cheveux gris naturels à 90 % de blancs** | Gris légèrement doré | 34,77 | 1,42 | 2,23 |
| **cheveux gris naturels permanentes à 90 % de blancs** | Gris légèrement doré | 22,43 | 0,96 | -0,31 |

## Revendications

1. Composition de teinture des fibres kératiniques comprenant, dans un milieu de teinture approprié,
• au moins un coupleur de formule (l) 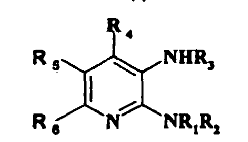 dans laquelle
- R3 et R4 représentent, indépendamment un atome d'hydrogène ; un radical alkyle linéaire ou ramifié de 1 à 6 atomes de carbone, éventuellement substitué par un ou plusieurs atomes d'halogène, un ou plusieurs radicaux hydroxy, amino, mono, di ou tri alkyle(C1-C4)amino, aryle, alcoxyC₁-C₃ ou hétérocycle ; un radical arylC₆-C₁₈, éventuellement substitué par un ou plusieurs radicaux amino, hydroxy, alcoxy(C₁-C₃) ou alkyl(C₁-C₆) ; un radical alcényle en C₂-C₆ : un radical carboxyalkyl(C₁-C₆) ou un radical alkylthio(C₁-C₆) ; et
- R₁ et R₂, ensemble, forment avec l'atome d'azote auquel ils sont rattachés un cycle pyrrolidine, pipéridine, homopipéridine, imidazoline, pyrazolidine, piperazine.
- R₅ représente un atome d'hydrogène ; un radical hydroxy ; alcoxy ; aryloxy ; un atome d'halogène ; un radical -OSO₂R ; un radical -OCOR ; un radical trifluoroalcoxy en C₁-C₃ ;
- R6 représente un radical hydroxy ; OR ; aryloxy ; un atome d'halogène ; un radical -OSO₂R ; un radical -OCOR ; un radical trifluoroalcoxy en C₁-C₃ ;
- R représente un radical alkyle en C₁-C₆ pouvant être substitué par un radical hydroxy, amino ou alcoxy en C₁-C₄ ;
• au moins une base d'oxydation hétérocyclique choisie parmi les pyridines non condensés, les pyrazoles non condensés, les pyrazolopyrimidines condensées, les pyrazolotriazoles, les pyrazolotétrazoles, les pyrazolopyridazines, les pyrazolothiazoles, les pyrazoloimidazoles, les pyrazoloquinolines, les pyrazolopyrazoles, les aminopyrazolones, les aminopyrolidines, les aminopyrazolines, les monoaminoquinoleines, les aminoindazoles, les diamino-uraciles, les hydrazones, les aminoindolénines, les diamino ou triaminoquinoleines, les dérivés de julolidine ou de lilolidine, les pyrazolobenzimidazoles.

2. Composition selon la revendication 1 dans laquelle les diamino-uraciles sont choisis parmi les composés 5,6-diamino -uracile, 5,6-diamino-2-thio -uracile, 5,6-diamino-3-méthyl -uracile, 5-amino-3-méthyl-6-méthylamino -uracile, 5-amino-3-méthyl-6-bétahydroxyéthylamino -uracile, 5-amino-3-méthyl-8-benzylamino uracile, 5-amino-3-méthyl-6-phénylamino -uracile, 5,8-diamino-1-phényl-uracile, 5,6-diamino-1,3-diméthyl-uracile, 5-amino-1,3-diméthyl-6-méthylamino -uracile, 5-amino-1,3-diméthyl-6-bétahydroxyméthylamino -uracile, 5-amino-1,3-diméthyl-6-benzyllamino - uracile, 5-amino-1,3-diméthyl-6-phényllamino -uracile, 5-amino-1,3-diméthyl-6-diméthylamino - uracile.

3. Composition selon la revendication 1 dans laquelle les aminoindolénines sont choisies parmi les composés 2-méthyl-5-aminoindolénine, 1-bétahydroxyéthyl-2-méthyl-5-aminoindolénine.

4. Composition selon la revendication 1 dans laquelle les mono ou diaminotétrahydroquinoléines sont choisies les composés parmi 5-amino-1,2,3,4-tétrahydroquinoléine, 5-amino-7-chloro-pipéridino-1,2,3,4-tétrahydroquinoléine, 5-amino-7-chloro-8-morpholino-1,2,3,4-tétrahydroquinoléine, 5,7-diamino-6-méthyl-8-hydroxy-1,2,3,4-tétrahydroquinoléine, 5-amino-8-méthoxy-1,2,3,4-tétrahydroquinoléine, 5-amino-7-chloro-8-diméthylamino-1,2,3,4-tétrahydroquinoléine.

5. Composition selon la revendication 1 dans laquelle les diaminoquinoléines sont choisies parmi les composés 5,7-diamino-6-méthyl-8-hydroxyquinoléine et 5,7-diamino-2-méthyl-8-hydroxyquinoléine.

6. Composition selon la revendication 1 dans laquelle les triaminoquinoléines sont choisies parmi les composés 5,7-diamino-8-méthylaminoquinoléine, 5,7-diamino-8-diméthylaminoquinoléine, 5,7-diamino-8-morpholinoquinoléine, 5,7-diamino-8-bétahydroxyéthylaminoquinoléine, 5,7,8-triaminoquinoléine.

7. Composition selon la revendication 1 dans laquelle les aminopyrazolones sont choisies parmi l'acide 1-phényl-4-aminopyrazolone-3-carbonique, l'amide de l'acide 1-phényl-4-aminopyrazolone-3-carbonique, l'ester éthylique de l'acide 1-phényl-4-aminopyrazolone-3-carbonique, la 1-phényl-3-méthyl-4-aminopyrazolone.

8. Composition selon la revendication 1 dans laquelle les aminopyrazolones sont choisis parmi les composés 3-amino-pyrazolone, 1-phényl-3-amino-pyrazolone, 1-phényl-3-hydroxy-pyrazolone.

9. Composition selon la revendication 1 dans laquelle les pyrazolothiazoles sont choisis parmi les composés 3-amino-2-méthyl-pyrazolo[3,2-b]thiazole, 3-amino-pyrazolo[3,2-b]thiazole, 3-amino-2,5-diméthyl-6-phénylpyrazolo[3,2-b]thiazole.

10. Composition selon la revendication 1 dans laquelle les pyrazolotriazoles sont choisis parmi 3-amino-4-méthyl-6-méthylthio-2-phényl-pyrazolo[3,2-c]-s-triazole, 3-amino-2,4,6-triméthyl-pyrazolo[3,2-c]-s-triazole, 3-amino-4,6-diméthyl-pyrazolo[3,2-c]-s-triazole.

11. Composition selon la revendication 1 dans laquelle les pyrazoloimidazoles sont choisis parmi les composés 3-amino-4-benzyl-pyrazolo[1,5-a]imidazole, 3-amino-2,4-diméthylpyrazolo[1,5-a]imidazole, 3-amino-4-méthyl-pyrazolo[1,5-a]imidazole.

12. Composition selon la revendication 1 dans laquelle la pyrazoloquinoline est la 3-amino-2-phényl-pyrazolo[1,5-a]quinoline.

13. Composition selon la revendication 1 dans laquelle la pyrazolopyridazine est la 3-amino-pyrazolo[1,5-b]pyridazine.

14. Composition selon la revendication 1 dans laquelle les dérivés de julolidine ou de lilolidine sont choisis parmi les composés 9-aminojulolidine, 9-amino-8-méthyljulolidine, 9-amino-8,10-diméthyljulolidine, 8-aminolilolidine.

15. Composition selon la revendication 1 dans laquelle les hydrazones sont choisis parmi les composés N-méthyl-pyridone-4-hydrazone, N-méthyl-thiazolone-hydrazone N-méthyl-thiazolone-2-hydrazone, N,N-diméthyl-benzimidazolone-hydrazone, N-méthyl-pyridone-2-hydrazone, N-méthyl-benzothiazolone-2-hydrazone, 1,2-diméthyl-indazolone-3-hydrazone, 1,2,6-triméthyl-pyridone-4-hydrazone, 1-méthyl-quinolone-2-hydrazone, 1,2,6-triméthyl-3-nitropyridone-4-hydrazone, 1,2,6-triméthyl-3-aminopyridone-4-hydrazone, N-méthyl-cyclohexènothiazolone-hydrazone, 1,2,5-triméthyl-pyrazolone-3-hydrazone, 1,2-diméthyl-indazolone-3-hydrazone, 1,2-diméthyl-5-chloro-indazolone-3-hydrazone, 1-méthyl-2-éthyl-5-nitro-indazolone-3-hydrazone, N-méthyl-quinolone-4-hydrazone, N-méthyl-benzothiazolone-2-omégabenzènesulfonyl-hydrazone.

16. Composition selon la revendication 1 dans laquelle l'aminopyrazoline est choisi parmi 1-(4'-aminophényl)-3-aminopyrazoline, 1-(4'-hydroxyphényl)-3-aminopyrazoline.

17. Composition selon la revendication 1 dans laquelle l'aminoindazole est choisi parmi 4,7-diamino-5-méthylindazole, 4,7-diamino-5,6-diméthylindazole, 6,7-diaminoindazole, 6-hydroxy-7-aminoindazole, 1-éthyl-6-hydroxy-7-aminoindazole, 6-aminoindazole, 5,6-diaminoindazole.

18. Composition selon l'une quelconque des revendications précédentes
dans laquelle la base d'oxydation est choisie parmi les pyridines non condensées, les pyrazoles non condensées, les pyrazolopyrimidines, les pyrazolotriazoles.

19. Composition selon la reendication 18 laquelle la base d'oxydation hétérocyclique est choisi parmi la 2,5-diamino pyridine, le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(p-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, 1,3-bis-(4,5-diaminopyrazol-1-yl)propane, 1,3-bis-(4,5-diaminopyrazol-1-yl)propane-2-amine, 2, 2'-bis-(4,5-diaminopyrazol-1-yl)diéthylamine, 1,3-bis-(4,5-diaminopyrazol-1-yl)propane-2-ol, 1,3-bis-(4,5-diaminopyrazol-1-yl)propane-2-ol, 2, 2'-bis-(4,5-diaminopyrazol-1-yl)diéthylether, 2, 2'-bis-(4,5-diaminopyrazol-1-yl)diéthylthioéther, 2, 2'-bis-(4,5-diaminopyrazol-1-yl)diéthylméthylamine, 2, 2'-bis-(4,5-diaminopyrazol-1-yl)diéthyldiméthylamonium, la 7-amino 3-méthylpyrazole[3,2,c]-1,2,4-triazole, la 7-amino 2-méthyl pyrazolo [1,5-b]1,2,4-triazole, la pyrazolo-[1,5-a]-pyrimidine-3,7-diamine ; la 2,5-diméthyl pyrazolo-[1,5-a]-pyrimidine-3,7-diamine ; la 3-amino-7-β-hydroxyéthylamino-5-méthyl-pyrazolo-[1,5-a]-pyrimidine; le 2-(7-amino pyrazolo-[1,5-a]-pyrimidin-3-ylamino)-éthanol ; ; la 5,6-diméthyl pyrazolo-[1,5-a]pyrimidin-3,7-diamine ; la 2, 5, N-7, N-7-tétraméthyl pyrazolo-[1,5-a]-pyrimidine-3,7-diamine ; la N-7-méthyl N-7-éthyl pyrazolo[1,5-a]-pyrimidine-3,7-diamine et leurs sels d'addition et leurs formes tautomères, lorsqu'il existe un équilibre tautomérique.

20. Composition selon l'une quelconque des revendications 1 à 19 dans laquelle le coupleur de peut être représenté par la formule (l') 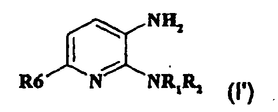 dans laquelle R₁, R₂ et R₆ sont tels que définis précédemment.

21. Composition selon la revendication 20 dans laquelle R6 est OR.

22. Composition selon l'une quelconque des revendications 1 à 21 dans laquelle le coupleur pyridinique est choisi parmi les composés 6-méthoxy 3-amino 2-pyrrolidinylpyridine, méthoxy 3-amino 2-(3'acétamidopyrrolidinyl)pyridine, 6-méthoxy 3-amino 2-(2'5'diméthylpyrrolidinyl)pyridine, 6-méthoxy 3-amino 2-morpholinopyridine, 6-méthoxy 3-amino 2-(2'-méthylpyrrolidinyl)pyridine, 6-méthoxy 3-amino 2-pipérazinylpyridine, 6-méthoxy 3-amino 2-pyridinylpyridine, méthoxy 3-amino 2-pyrrolidinylpyridine, 6-méthoxy 3-amino 2-(2'-méthylpyridinyl)pyridine, 6-méthoxy 3-amino 2-(2'-hydraxyéthylpyridinyl)pyridine, 6-méthoxy 3-amino 2-(2'-pyrrolidinyléthyl)aminopyridine, 6-méthoxy 3-amino2-(3'imidazolinylpropyl)aminopyridine, 6-méthoxy-3-amino 2-[3'-(3"-méthylimidazolium)propyl)aminopyridine.

23. Composition selon la revendication 22 dans laquelle le coupleur pyridinique est choisi parmi les composés, 6-méthoxy 3-amino 2-pyrrolidinylpyridine, 6-méthoxy 3-amino 2-(2'-méthylpyrrolidinyl)pyridine, 6-méthoxy 3-amino 2-(2'-méthylpyridinyl)pyridine, 6-méthoxy 3-amino 2-(2'-hydroxyéthylpyridinyl)pyridine.

24. Composition selon l'une quelconque des revendications précédentes comprenant une base d'oxydation additionnelle choisie parmi les paraphénylènediamines, les bis-phényialkylènediamines, les para-aminophénols, les ortho-aminophénols et leurs sels d'addition.

25. Composition selon l'une quelconque des revendications précédentes comprenant un coupleur choisi parmi les métaphénylènediamines, les méta-aminophénols, les métadiphénols, les coupleurs naphtaléniques, les coupleurs hétérocycliques autres que les coupleurs de formule (l) et leurs sels d'addition.

26. Composition selon l'une quelconque des revendications précédentes dans laquelle la quantité de chacune des bases d'oxydation est comprise entre 0,001 à 10 % en poids environ du poids total de la composition tinctoriale.

27. Composition selon l'une quelconque des revendications précédentes
dans laquelle la quantité de chacun des coupleurs est comprise entre 0,001 et 10 % en poids environ du poids total de la composition tinctoriale.

28. Composition selon l'une quelconque des revendications 1 à 27 contenant de plus un agent oxydant.

29. Composition selon l'une quelconque des revendications 1 à 27 présentant un pH compris entre 3 et 12, de préférence entre 5 et 8.

30. Procédé de teinture d'oxydation des fibres kératiniques, **caractérisé en ce qu'**on applique sur les fibres une composition tinctoriale telle que définie dans l'une quelconque des revendications 1 à 27 en présence d'un agent oxydant pendant un temps suffisant pour développer la couleur désirée.

31. Procédé selon la revendication 30 dans lequel le pH de la composition prête à l'emploi est inférieur à 9.

32. Procédé selon la revendication 31 dans lequel le pH de la composition prête à l'emploi est inférieur ou égal à 7.

33. Procédé selon l'une quelconque des revendications 30 à 32 dans lequel l'agent oxydant est l'oxygène de l'air ou un agent choisi parmi le peroxyde d'hydrogène, le peroxyde d'urée, les bromates de métaux alcalins, les persels, les peracides et les enzymes oxydases.

34. Dispositif à plusieurs compartiments dans lequel un premier compartiment contient une composition tinctoriale telle que définie à l'une quelconque des revendications 1 à 27 et un deuxième compartiment contient un agent oxydant.

35. Kit pour la coloration des cheveux comprenant d'une part une composition tinctoriale telle que défini à la revendication 1 à 27 et d'autre part une composition contenant un agent oxydant.

36. Utilisation de la composition définie aux revendications 1 à 28 pour la teinture de fibres kératiniques.

37. Utilisation selon la revendication 36 pour la teinture des fibres kératiniques dans des nuances grise à noires.

## Claims

1. Composition for dyeing keratin fibres, comprising, in a suitable dyeing medium:
• at least one coupler of formula (I) 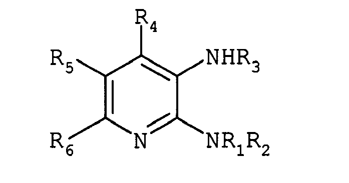 in which:
- R₃ and R₄ independently represent a hydrogen atom; a linear or branched alkyl radical of 1 to 6 carbon atoms, optionally substituted with one or more halogen atoms, or one or more hydroxyl, amino, mono-, di- or tri (C₁-C₄) alkylamino, aryl, C₁-C₃ alkoxy or heterocyclic radicals; a C₆-C₁₈ aryl radical, optionally substituted with one or more amino, hydroxyl, (C₁-C₃) alkoxy or (C₁-C₆) alkyl radicals; a C₂-C₆ alkenyl radical; a carboxy(C₁-C₆)alkyl radical or a (C₁-C₆)alkylthio radical; and
- R₁ and R₂ together form, with the nitrogen atom to which they are attached, a pyrrolidine, piperidine, homopiperidine, imidazoline, pyrazolidine or piperazine ring;
- R₅ represents a hydrogen atom; a hydroxyl radical; alkoxy; aryloxy; a halogen atom; a radical -OSO₂R; a radical -OCOR; a C₁-C₃ trifluoroalkoxy radical;
- R6 represents a hydroxyl radical; OR; aryloxy; a halogen atom; a radical -OSO₂R; a radical -OCOR; a C₁-C₃ trifluoroalkoxy radical;
- R represents a C₁-C₆ alkyl radical that may be substituted with a hydroxyl, amino or C₁-C₄ alkoxy radical;
• at least one heterocyclic oxidation base chosen from non-fused pyridines, non-fused pyrazoles, fused pyrazolopyrimidines, pyrazolotriazoles, pyrazolotetrazoles, pyrazolopyridazines, pyrazolothiazoles, pyrazoloimidazoles, pyrazoloquinolines, pyrazolopyrazoles, aminopyrazolones, aminopyrrolidines, aminopyrazolines, monoaminoquinolines, aminoindazoles, diaminouracils, hydrazones, aminoindolenines, diamino- or triaminoquinolines, julolidine or lilolidine derivatives, and pyrazolobenzimidazoles.

2. Composition according to Claim 1, in which the diaminouracils are chosen from the compounds 5,6-diaminouracil, 5,6-diamino-2-thiouracil, 5,6-diamino-3-methyluracil, 5-amino-3-methyl-6-methylaminouracil, 5-amino-3-methyl-6β-hydroxyethylaminouracil, 5-amino-3-methyl-6-benzylaminouracil, 5-amino-3-methyl-6-phenyl-aminouracil, 5,6-diamino-1-phenyluracil, 5,6-diamino-1,3-dimethyluracil, 5-amino-1,3-dimethyl-6-methyl-aminouracil, 5-amino-1,3-dimethyl-6β-hydroxymethyl-aminouracil, 5-amino-1,3-dimethyl-6-benzylaminouracil, 5-amino-1,3-dimethyl-6-phenylaminouracil and 5-amino-1,3-dimethyl-6-dimethylaminouracil.

3. Composition according to Claim 1, in which the aminoindolenines are chosen from the compounds 2-methyl-5-aminoindolenine and 1β-hydroxyethyl-2-methyl-5-aminoindolenine.

4. Composition according to Claim 1, in which the mono- or diaminotetrahydroquinolines are chosen from the compounds 5-amino-1,2,3,4-tetrahydroquinoline, 5-amino-7-chloro-8-piperidino-1,2,3,4-tetrahydroquinoline, 5-amino-7-chloro-8-morpholino-1,2,3,4-tetrahydroquinoline, 5,7-diamino-6-methyl-8-hydroxy-1,2,3,4-tetrahydroquinoline, 5-amino-8-methoxy-1,2,3,4-tetrahydroquinoline and 5-amino-7-chloro-8-dimethylamino-1,2,3,4-tetrahydroquinoline.

5. Composition according to Claim 1, in which the diaminoquinolines are chosen from the compounds 5,7-diamino-6-methyl-8-hydroxyquinoline and 5,7-diamino-2-methyl-8-hydroxyquinoline.

6. Composition according to Claim 1, in which the triaminoquinolines are chosen from the compounds 5,7-diamino-8-methylaminoquinoline, 5,7-diamino-8-dimethyl-aminoquinoline, 5,7-diamino-8-morpholinoquinoline, 5,7-diamino-8β-hydroxyethylaminoquinoline and 5,7,8-triaminoquinoline.

7. Composition according to Claim 1, in which the aminopyrazolones are chosen from 1-phenyl-4-amino-pyrazolone-3-carbonic acid, 1-phenyl-4-aminopyrazolone-3-carbonic acid amide, ethyl 1-phenyl-4-amino-pyrazolone-3-carbonate and 1-phenyl-3-methyl-4-amino-pyrazolone.

8. Composition according to Claim 1, in which the aminopyrazolones are chosen from the compounds 3-aminopyrazolone, 1-phenyl-3-aminopyrazolone and 1-phenyl-3-hydroxypyrazolone.

9. Composition according to Claim 1, in which the pyrazolothiazoles are chosen from the compounds 3-amino-2-methylpyrazolo[3,2-b]thiazole, 3-amino-pyrazolo[3,2-b]thiazole and 3-amino-2,5-dimethyl-6-phenylpyrazolo[3,2-b]thiazole.

10. Composition according to Claim 1, in which the pyrazolotriazoles are chosen from 3-amino-4-methyl-6-methylthio-2-phenylpyrazolo[3,2-c]-s-triazole, 3-amino-2,4,6-trimethylpyrazolo[3,2-c]-s-triazole and 3-amino-4,6-dimethylpyrazolo[3,2-c]-s-triazole.

11. Composition according to Claim 1, in which the pyrazoloimidazoles are chosen from the compounds 3-amino-4-benzylpyrazolo[1,5-a]imidazole, 3-amino-2,4-dimethylpyrazolo[1,5-a]imidazole and 3-amino-4-methyl-pyrazolo[1,5-a]imidazole.

12. Composition according to Claim 1, in which the pyrazoloquinoline is 3-amino-2-phenylpyrazolo[1,5-a]-quinoline.

13. Composition according to Claim 1, in which the pyrazolopyridazine is 3-aminopyrazolo[1,5-b]pyridazine.

14. Composition according to Claim 1, in which the julolidine or lilolidine derivatives are chosen from the compounds 9-aminojulolidine, 9-amino-8-methyl-julolidine, 9-amino-8,10-dimethyljulolidine and 8-aminolilolidine.

15. Composition according to Claim 1, in which the hydrazones are chosen from the compounds N-methylpyridone 4-hydrazone, N-methylthiazolone hydrazone, N-methylthiazolone 2-hydrazone, N,N-dimethylbenzimidazolone hydrazone, N-methylpyridone 2-hydrazone, N-methylbenzothiazolone 2-hydrazone, 1,2-dimethylindazolone 3-hydrazone, 1,2,6-trimethylpyridone 4-hydrazone, 1-methylquinolone 2-hydrazone, 1,2,6-trimethyl-3-nitropyridone 4-hydrazone, 1,2,6-trimethyl-3-aminopyridone 4-hydrazone, N-methylcyclohexenothiazolone hydrazone, 1,2,5-trimethylpyrazolone 3-hydrazone, 1,2-dimethylindazolone 3-hydrazone, 1,2-dimethyl-5-chloroindazolone 3-hydrazone, 1-methyl-2-ethyl-5-nitroindazolone 3-hydrazone, N-methylquinolone 4-hydrazone and N-methylbenzothiazolone 2ω-benzene-sulfonylhydrazone.

16. Composition according to Claim 1, in which the aminopyrazoline is chosen from 1-(4'-aminophenyl)-3-aminopyrazoline and 1-(4'-hydroxyphenyl)-3-aminopyrazoline.

17. Composition according to Claim 1, in which the aminoindazole is chosen from 4,7-diamino-5-methylindazole, 4,7-diamino-5,6-dimethylindazole, 6,7-diaminoindazole, 6-hydroxy-7-aminoindazole, 1-ethyl-6-hydroxy-7-aminoindazole, 6-aminoindazole and 5,6-diaminoindazole.

18. Composition according to any one of the preceding claims, in which the oxidation base is chosen from non-fused pyridines, non-fused pyrazoles, pyrazolopyrimidines and pyrazolotriazoles.

19. Composition according to Claim 18, in which the heterocyclic oxidation base is chosen from 2,5-diaminopyridine, 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-l-methyl-3-phenylpyrazole, 4-amino-1,3-dimethyl-5-hydrazinopyrazole, 1-benzyl-4,5-diamino-3-methylpyrazole, 4,5-diamino-3-tert-butyl-l-methylpyrazole, 4,5-diamino-l-tert-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methyl-aminopyrazole, 3,5-diamino-4-(β-hydroxyethyl)amino-1-methylpyrazole, 1,3-bis(4,5-diaminopyrazol-1-yl)-propane, 1,3-bis(4,5-diaminopyrazol-1-yl)propan-2-amine, 2,2'-bis(4,5-diaminopyrazol-1-yl)diethylamine, 1,3-bis(4,5-diaminopyrazol-1-yl)propan-2-ol, 1,3-bis(4,5-diaminopyrazol-1-yl)propan-2-ol, 2,2'-bis(4,5-diaminopyrazol-1-yl)diethyl ether, 2,2'-bis(4,5-diaminopyrazol-1-yl)diethyl thioether, 2,2'-bis(4,5-diaminopyrazol-1-yl)diethylmethylamine, 2,2'-bis(4,5-diaminopyrazol-1-yl)diethyldimethylammonium, 7-amino-3-methylpyrazole[3,2-c]-1,2,4-triazole, 7-amino-2-methyl-pyrazolo[1,5-b]-1,2,4-triazole, pyrazolo[1,5-a]pyrimidine-3,7-diamine; 2,5-dimethylpyrazolo[1,5-a]pyrimidine-3,7-diamine; 3-amino-7β-hydroxyethylamino-5-methylpyrazolo[1,5-a]pyrimidine; 2-(7-aminopyrazolo-[1,5-a]pyrimidin-3-ylamino)ethanol; 5,6-dimethyl-pyrazolo[1,5-a]pyrimidine-3,7-diamine; 2,5,N-7,N-7-tetramethylpyrazolo[1,5-a]pyrimidine-3,7-diamine; N-7-methyl-N-7-ethylpyrazolo[1,5-a]pyrimidine-3,7-diamine, and the addition salts thereof, and the tautomeric forms thereof, when a tautomeric equilibrium exists.

20. Composition according to any one of Claims 1 to 19, in which the coupler may be represented by formula (I'): 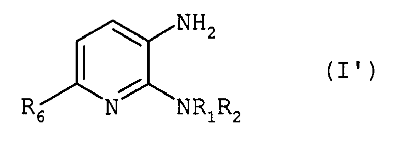 in which R₁, R₂ and R₆ are as defined above.

21. Composition according to Claim 20, in which R₆ is OR.

22. Composition according to any one of Claims 1 to 21, in which the pyridine-based coupler is chosen from the compounds 6-methoxy-3-amino-2-pyrrolidinylpyridine, 6-methoxy-3-amino-2-(3'-acetamidopyrrolidinyl)pyridine, 6-methoxy-3-amino-2-(2',5'-dimethylpyrrolidinyl)-pyridine, 6-methoxy-3-amino-2-morpholinopyridine, 6-methoxy-3-amino-2-(2'-methylpyrrolidinyl)pyridine, 6-methoxy-3-amino-2-piperazinylpyridine, 6-methoxy-3-amino-2-pyridylpyridine, 6-methoxy-3-amino-2-pyrrolidinylpyridine, 6-methoxy-3-amino-2-(2'-methylpyridyl)-pyridines 6-methoxy-3-amino-2-(2'-hydroxyethylpyridyl)-pyridine, 6-methoxy-3-amino-2-(2'-pyrrolidinylethyl)-aminopyridine, 6-methoxy-3-amino-2-(3'-imidazolinyl-propyl)aminopyridine and 6-methoxy-3-amino-2-[3'-(3"-methylimidazolium)propyl]aminopyridine.

23. Composition according to Claim 22, in which the pyridine-based coupler is chosen from the compounds 6-methoxy-3-amino-2-pyrrolidinylpyridine, 6-methoxy-3-amino-2-(2'-methylpyrrolidinyl)pyridine, 6-methoxy-3-amino-2-(2'-methylpyridyl)pyridine and 6-methoxy-3-amino-2-(2'-hydroxyethylpyridyl)pyridine.

24. Composition according to any one of the preceding claims, comprising an additional oxidation base chosen from para-phenylenediamines, bis(phenyl)alkylenediamines, para-aminophenols and ortho-aminophenols, and the addition salts thereof.

25. Composition according to any one of the preceding claims, comprising a coupler chosen from meta-phenylenediamines, meta-aminophenols, meta-diphenols, naphthalene-based couplers and heterocyclic couplers other than the couplers of formula (I), and the addition salts thereof.

26. Composition according to any one of the preceding claims, in which the amount of each of the oxidation bases is between 0.001% and 10% by weight approximately relative to the total weight of the dye composition.

27. Composition according to any one of the preceding claims, in which the amount of each of the couplers is between 0.001% and 10% by weight approximately relative to the total weight of the dye composition.

28. Composition according to any one of Claims 1 to 27, also containing an oxidizing agent.

29. Composition pH according to any one 12 of Claims 1 to 27, having a pH of between 3 and 12 and preferably between 5 and 8.

30. Process for the oxidation dyeing of keratin fibres, **characterized in that** a dye composition as defined in any one of Claims 1 to 27 is applied to the fibres in the presence of an oxidizing agent, for a time that is sufficient to develop the desired colour.

31. Process according to Claim 30, in which the pH of the ready-to-use composition is less than 9.

32. Process according to Claim 31, in which the pH of the ready-to-use composition is less than or equal to 7.

33. Process according to any one of Claims 30 to 32, in which the oxidizing agent is atmospheric oxygen or an agent chosen from hydrogen peroxide, urea peroxide, alkali metal bromates, persalts, peracids and oxidase enzymes.

34. Multi-compartment device in which a first compartment contains a dye composition as defined in any one of Claims 1 to 27 and a second compartment contains an oxidizing agent.

35. Hair-dyeing kit comprising, firstly, a dye composition as defined in Claims 1 to 27, and, secondly, a composition containing an oxidizing agent.

36. Use of the composition defined in Claims 1 to 28, for dyeing keratin fibres.

37. Use according to Claim 36, for dyeing keratin fibres in grey to black shades.

## Patentansprüche

1. Zusammensetzung zum Färben von Keratinfasern, die in einem zum Färben geeigneten Medium enthält:
• mindestens einen Kuppler der Formel (I): 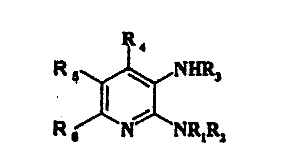 in der Formel:
- R₃ und R₄ bedeuten unabhängig voneinander ein Wasserstoffatom; eine geradkettige oder verzweigte Alkylgruppe mit 1 bis 6 Kohlenstoffatomen, die gegebenenfalls mit einem oder mehreren Halogenatomen, einer oder mehreren Gruppen Hydroxy, Amino, Mono-, Di- oder Trialkyl(C₁-₄)-amino, Aryl, Alkoxy(C₁₋₃) oder einem oder mehreren Heterocyclen substituiert ist; eine Aryl(C₆₋₁₈)gruppe, die gegebenenfalls mit einer oder mehreren Gruppen Amino, Hydroxy, Alkoxy(C₁₋₃) oder Alkyl(C₁₋₆) substituiert ist; eine Alkenyl(C₂₋₆)gruppe; eine Carboxyalkyl(C₁₋₆)gruppe oder eine Alkylthio(C₁₋₆)gruppe; und
- R₁ und R₂ bilden gemeinsam mit dem Stickstoffatom, an das sie gebunden sind, einen Pyrrolidinring, Piperidinring, Homopiperidinring, Imidazolinring, Pyrazolidinring, Piperazinring;
- R₅ bedeutet ein Wasserstoffatom; eine Hydroxygruppe; Alkoxy; Aryloxy; ein Halogenatom; eine Gruppe -OSO₂R; eine Gruppe -OCOR; eine Trifluoralkoxy(C₁₋₃)gruppe;
- R₆ bedeutet eine Hydroxygruppe; OR; Aryloxy; ein Halogenatom; eine Gruppe -OSO₂R; eine Gruppe -OCOR; eine Trifluoralkoxy(C₁₋₃)gruppe;
- R bedeutet eine Alkyl(C₁₋₆)gruppe, die mit einer Gruppe Hydroxy, Amino oder Alkoxy mit 1 bis 4 Kohlenstoffatomen substituiert sein kann;
• mindestens eine heterocyclische Oxidationsbase, die unter den nichtkondensierten Pyridinen, nichtkondensierten Pyrazolen, kondensierten Pyrazolopyrimidinen, Pyrazolotriazolen, Pyrazolotetrazolen, Pyrazolopyridazinen, Pyrazolothiazolen, Pyrazoloimidazolen, Pyrazolochinolinen, Pyrazolopyrazolen, Aminopyrazolonen, Aminopyrrolidinen, Aminopyrazolinen, Monoaminochinolinen, Aminoindazolen, Diaminouracilen, Hydrazonen, Aminoindoleninen, Diamino- oder Triaminochinolinen, Julolidinderivaten oder Lilolidinderivaten, Pyrazolobenzimidazolen ausgewählt ist.

2. Zusammensetzung nach Anspruch 1, wobei die Diaminouracile ausgewählt sind unter den Verbindungen 5,6-Diamino-uracil, 5,6-Diamino-2-thio-uracil, 5,6-Diamino-3-methyl-uracil, 5-Amino-3-methyl-6-methylamino-uracil, 5-Amino-3-methyl-6-betahydroxyethylamino-uracil, 5-Amino-3-methyl-6-benzyl-amino-uracil, 5-Amino-3-methyl-6-phenylamino-uracil, 5,6-Diamino-1-phenyl-uracil, 5,6-Diamino-1,3-dimethyl-uracil, 5-Amino-1,3-dimethyl-6-methylamino-uracil, 5-Amino-1,3-dimethyl-6-betahydroxymethylamino-uracil, 5-Amino-1,3-dimethyl-6-benzylamino-uracil, 5-Amino-1,3-dimethyl-6-phenylamino-uracil, 5-Amino-1,3-dimethyl-6-dimethylamino-uracil.

3. Zusammensetzung nach Anspruch 1, wobei die Aminoindolenine unter den Verbindungen 2-Methyl-5-aminoindolenin, 1-beta-Hydroxyethyl-2-methyl-5-aminoindolenin ausgewählt sind.

4. Zusammensetzung nach Anspruch 1, wobei die Mono- oder Diaminotetrahydrochinoline unter den Verbindungen 5-Amino-1,2,3,4-tetrahydrochinolin, 5-Amino-7-chlor-8-piperidino-1,2,3,4-tetrahydrochinolin, 5-Amino-7-chlor-8-morpholino-1,2,3,4-tetrahydrochinolin, 5,7-Diamino-6-methyl-8-hydroxy-1,2,3,4-tetrahydrochinolin, 5-Amino-8-methoxy-1,2,3,4-tetra-hydrochinolin, 5-Amino-7-chloro-8-dimethylamino-1,2,3,4-tetrahydrochinolin ausgewählt sind.

5. Zusammensetzung nach Anspruch 1, wobei die Diaminochinoline unter den Verbindungen 5,7-Diamino-6-methyl-8-hydroxychinolin und 5,7-Diamino-2-methyl-8-hydroxychinolin ausgewählt sind.

6. Zusammensetzung nach Anspruch 1, wobei die Triaminochinoline unter den Verbindungen 5,7-Diamino-8-methylaminochinolin, 5,7-Diamino-8-dimethylaminochinolin, 5,7-Diamino-8-morpholinochinolin, 5,7-Diamino-8-betahydroxyethylaminochinolin, 5,7,8-Triaminochinolin ausgewählt sind.

7. Zusammensetzung nach Anspruch 1, bei der die Aminopyrazolone unter 1-Phenyl-4-aminopyrazolon-3-carbonsäure, dem Amid von 1-Phenyl-4-aminopyrazolon-3-carbonsäure, dem Ethylester von 1-Phenyl-4-aminopyrazolon-3-carbonsäure, 1-Phenyl-3-methyl-4-aminopyrazolon ausgewählt sind.

8. Zusammensetzung nach Anspruch 1, bei der Aminopyrazolone unter den Verbindungen 3-Aminopyrazolon, 1-Phenyl-3-aminopyrazolon und 1-Phenyl-3-hydroxypyrazolon ausgewählt sind.

9. Zusammensetzung nach Anspruch 1, bei der die Pyrazolothiazole unter den Verbindungen 3-Amino-2-methyl-pyrazolo[3,2-b]-thiazol, 3-Amino-pyrazolo[3,2-b]thiazol und 3-Amino-2,5-dimethyl-6-phenyl-pyrazolo[3,2-b]thiazol ausgewählt sind.

10. Zusammensetzung nach Anspruch 1, bei der die Pyrazolotriazole unter 3-Amino-5-methyl-6-methylthio-2-phenyl-pyrazolo[3,2-c]-s-triazol, 3-Amino-2,4,6-trimethyl-pyrazolo[3,2-c]-s-triazol und 3-Amino-4,6-dimethyl-pyrazolo[3,2-c]-s-triazol ausgewählt sind.

11. Zusammensetzung nach Anspruch 1, bei der die Pyrazoloimidazole unter den Verbindungen 3-Amino-4-benzyl-pyrazolo[1,5-a]-imidazol, 3-Amino-2,4-dimethyl-pyrazolo[1,5-a]imidazol, 3-Amino-4-methyl-pyrazolo[1,5-a]imidazol ausgewählt sind.

12. Zusammensetzung nach Anspruch 1, bei der das Pyrazolochinolin das 3-Amino-2-phenyl-pyrazolo[1,5-a]chinolin ist.

13. Zusammensetzung nach Anspruch 1, bei der das Pyrazolopyridazin das 3-Amino-pyrazolo[1,5-b]pyridazin ist.

14. Zusammensetzung nach Anspruch 1, bei der die Julolidinderivate oder Lilolidinderivate unter den Verbindungen 9-Aminojulolidin, 9-Amino-8-methyljulolidin, 9-Amino-8,10-dimethyljulolidin und 8-Aminolilolidin ausgewählt sind.

15. Zusammensetzung nach Anspruch 1, bei der die Hydrazone unter den Verbindungen N-Methyl-pyridon-4-hydrazon, N-Methylthiazolon-hydrazon, N-Methyl-thiazolon-2-hydrazon, N,N-Dimethyl-benzimidazolon-hydrazon, N-Methyl-pyridon-2-hydrazon, N-Methyl-benzothiazolon-2-hydrazon, 1,2-Dimethyl-indazolon-3-hydrazon, 1,2,6-Trimethyl-pyridon-4-hydrazon, 1-Methyl-chinolon-2-hydrazon, 1,2,6-Trimethyl-3-nitropyridon-4-hydrazon, 1,2,6-Trimethyl-3-aminopyridon-4-hydrazon, N-Methyl-cyclohexenothiazolon-hydrazon, 1,2,5-Trimethyl-pyrazolon-3-hydrazon, 1,2-Dimethyl-indazolon-3-hydrazon, 1,2-Dimethyl-5-chlorindazolon-3-hydrazon, 1-Methyl-2-ethyl-5-nitro-indazolon-3-hydrazon, N-Methyl-chinolon-4-hydrazon, N-Methyl-benzothiazolon-2-omegabenzolsulfonyl-hydrazon ausgewählt sind.

16. Zusammensetzung nach Anspruch 1, bei der das Aminopyrazolin unter 1-(4'-Aminophenyl)-3-aminopyrazolin und 1-(4'-Hydroxyphenyl)-3-aminopyrazolin ausgewählt ist.

17. Zusammensetzung nach Anspruch 1, bei der das Aminoindazol unter 4,7-Diamino-5-methylindazol 4,7-Diamino-5,6-dimethylindazol, 6,7-Diaminoindazol, 6-Hydroxy-7-aminoindazol, 1-Ethyl-6-hydroxy-7-aminoindazol, 6-Aminoindazol und 5,6-Diaminoindazol ausgewählt ist.

18. Zusammensetzung nach einem der vorhergehenden Ansprüche, bei der die Oxidationsbase unter den nichtkondensierten Pyridinen, nichtkondensierten Pyrazolen, Pyrazolopyrimidinen und Pyrazolotriazolen ausgewählt ist.

19. Zusammensetzung nach Anspruch 18, wobei die heterocyclische Oxidationsbase unter 2, 5-Diaminopyridin, 4, 5-Diamino-1-methylpyrazol 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert butyl-1-methylpyrazol, 4,5-Diamino-1-tert butyl-3-methylpyrazol, 4,5-Diamino-1-(β-hydroxyethyl)-3-methylpyrazol, 4, 5-Diamino-1-ethyl-3-methylyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol, 3,5-Diamino-4-(β-hydroxyethyl)-amino-1-methylpyrazol, 1,3-Bis(4,5-diaminopyrazol-1-yl)-propan, 1,3-Bis(4,5-diaminopyrazol-1-yl)-propan-2-amin, 2,2'-Bis(4,5-diaminopyrazol-1-yl)-diethylamin, 1,3-Bis(4,5-diaminopyrazol-1-yl)-propan-2-ol, 1,3-Bis(4,5-diaminopyrazol-1-yl)-propan-2-ol, 2,2'-Bis(4,5-diaminopyrazol-1-yl)-diethylether, 2,2'-Bis(4,5-diaminopyrazol-1-yl)-diethylthioether, 2,2'-Bis(4,5-diaminopyrazol-1-yl)-diethylmethylamin, 2,2'-Bis(Diaminopyrazol-1-yl)-diethyldimethylammonium, 7-Amino-3-methyl-pyrazol[3,2-c]-1,2,4-triazol, 7-Amino-2-methyl-pyrazolo[1,5-b]-1,2,4-triazol, Pyrazolo[1,5-a]pyrimidin-3,7-diamin; 2,5-Dimethyl-pyrazol[1,5-a]-pyrimidin-3,7-diamin; 3-Amino-7-β-hydroxyethylamino-5-methyl-pyrazolo[1,5-a]pyrimidin; 2-(7-Amino-pyrazolo[1,5-a]-pyrimidin-3-ylamino)-ethanol; 5,6-Dimethyl-pyrazolo[1,5-a]-pyrimidin-3,7-diamin; 2,5,N-7,N-7-Tetramethyl-pyrazolo[1,5-a]-pyrimidin-3,7-diamin, N-7-Methyl-N-7-ethyl-pyrazolo[1,5-a]-pyrimidin-3,7-diamin und deren Additionssalzen und, falls es ein tautomeres Gleichgewicht gibt, ihren tautomeren Formen ausgewählt ist.

20. Zusammensetzung nach einem der Ansprüche 1 bis 19, wobei der Kuppler die Formel (I') bedeuten kann, 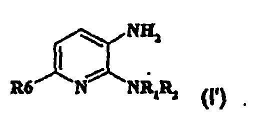 wobei die Gruppen R₁, R₂ und R₆ die oben angegebenen Bedeutungen aufweisen.

21. Zusammensetzung nach Anspruch 20, bei der R₆ OR bedeutet.

22. Zusammensetzung nach einem der Ansprüche 1 bis 21, bei der der Pyridinkuppler unter den Verbindungen 6-Methoxy-3-amino-2-pyrrolidinylpyridin, 6-Methoxy-3-amino-2-(3'-acetamidopyrrolidinyl)-pyridin, 6-Methoxy-3-amino-2-(2',5'-dimethylpyrrolidinyl)-pyridin, 6-Methoxy-3-amino-2-morpholinopyridin, 6-Methoxy-3-amino-2-(2'-methylpyrrolidinyl)-pyridin, 6-Methoxy-3-amino-2-piperazinylpyridin, 6-Methoxy-3-amino-2-pyridinylpyridin, 6-Methoxy-3-amino-2-pyrrolidinylpyridin, 6-Methoxy-3-amino-2-(2'-methylpyridinyl)-pyridin, 6-Methoxy-3-amino-2-(2'-hydroxyethylpyridinyl)-pyridin, 6-Methoxy-3-amino-2-(2'-pyrrolidinylethyl)-aminopyridin, 6-Methoxy-3-amino-2-(3'-imidazolinylpropyl)-aminopyridin, 6-Methoxy-3-amino-2-[3'-(3"-methylimidazolium)-propyl]-aminopyridin ausgewählt ist.

23. Zusammensetzung nach Anspruch 22, bei der der Pyridinkuppler unter den Verbindungen 6-Methoxy-3-amino-2-pyrrolidinylpyridin, 6-Methoxy-3-amino-2-(2'-methylpyrrolidinyl)-pyridin, 6-Methoxy-3-amino-2-(2'-methylpyridinyl)-pyridin, 6-Methoxy-3-amino-2-(2'-hydroxyethylpyridinyl)-pyridin ausgewählt ist.

24. Zusammensetzung nach einem der vorhergehenden Ansprüche, die eine ergänzende Oxidationsbase enthält, die unter den p-Phenylendiaminen, Bisphenylalkylendiaminen, para-Aminophenolen, ortho-Aminophenolen und ihren Additionssalzen ausgewählt ist.

25. Zusammensetzung nach einem der vorhergehenden Ansprüche, die einen Kuppler enthält, der unter den meta-Phenylendiaminen, meta-Aminophenolen, meta-Diphenolen, meta-Dihydroxybenzolen, Naphthalinkupplern, heterocyclischen Kupplern, die von den Kupplern der Formel (I) verschieden sind, und deren Additionssalzen ausgewählt ist.

26. Zusammensetzung nach einem der vorhergehenden Ansprüche, bei der der Mengenanteil jeder Oxidationsbase im Bereich von etwa 0,001 bis 10 Gew.-% des Gesamtgewichts der Farbmittelzusammensetzung liegt.

27. Zusammensetzung nach einem der vorhergehenden Ansprüche, bei der der Mengenanteil jedes Kupplers im Bereich von etwa 0,001 bis 10 Gew.-% des Gesamtgewichts der Farbmittelzusammensetzung liegt.

28. Zusammensetzung nach einem der Ansprüche 1 bis 27, die ferner ein Oxidationsmittel enthält.

29. Zusammensetzung nach einem der Ansprüche 1 bis 27, die einen pH-Wert im Bereich von 3 bis 12 und vorzugsweise 5 bis 8 aufweist.

30. Verfahren zum oxidativen Färben von Keratinfasern, **dadurch gekennzeichnet, dass** auf die Fasern eine Farbmittelzusammensetzung, wie es in einem der Ansprüche 1 bis 27 definiert ist, in Gegenwart eines Oxidationsmittels während einer Zeitspanne aufgebracht wird, die ausreicht, um die gewünschte Farbe zu bilden.

31. Verfahren nach Anspruch 30, bei dem der pH-Wert der gebrauchsfertigen Zusammensetzung unter 9 liegt.

32. Verfahren nach Anspruch 31, bei dem der pH-Wert der gebrauchsfertigen Zusammensetzung kleiner oder gleich 7 ist.

33. Verfahren nach einem der Ansprüche 30 bis 32, bei dem das Oxidationsmittel Luftsauerstoff oder ein Stoff ist, der unter Wasserstoffperoxid, Harnstoffperoxid, Alkalimetallbromaten, Salzen von Persäuren, Persäuren und Oxidasen ausgewählt ist.

34. Vorrichtung mit mehreren Abteilungen, bei der eine erste Abteilung eine Farbmittelzusammensetzung enthält, wie sie in einem der Ansprüche 1 bis 27 definiert ist, und eine zweite Abteilung ein Oxidationsmittel enthält.

35. Kit zum Färben der Haare, das einerseits eine Farbmittelzusammensetzung, wie sie in Ansprüche 1 bis 27 definiert ist, und andererseits eine Zusammensetzung, die ein Oxidationsmittel enthält, umfasst.

36. Verwendung der Zusammensetzung nach einem der Ansprüche 1 bis 28 zum Färben von Keratinfasern.

37. Verwendung nach Anspruch 36 zum Färben von Keratinfasern in grauen bis schwarzen Nuancen.
